# EUROPEAN PATENT SPECIFICATION

(11) **EP 0 538 912 B1**
(45) Date of publication and mention of the grant of the patent: **05.06.1996**
(21) Application number: 92121979.6
(22) Date of filing: 21.03.1991
(51) Int. Cl.: B01J 23/74, B01J 23/78, C07C 37/56

(54) **Use of a catalyst for producing phenol**
Verwendung eines Katalysators zur Herstellung von Phenol
Utilisation d'un catalyseur pour la production de phénol

(30) Priority: 17.04.1990 JP 99333/90; 30.07.1990 JP 199161/90; 11.12.1990 JP 401303/90; 27.12.1990 JP 408265/90; 27.12.1990 JP 408266/90; 27.12.1990 JP 408267/90; 27.12.1990 JP 408268/90
(43) Date of publication of application: 28.04.1993
(62) Divisional of application: 91104471.7
(73) Proprietor: NKK CORPORATION, Tokyo (JP)
(72) Inventor: Miki, Jun, c/o NKK CORPORATION, Tokyo (JP); Suzuki, Toshifumi, c/o NKK CORPORATION, Tokyo (JP); Shikada, Tsutomu, c/o NKK CORPORATION, Tokyo (JP); Tate, Kazuhiko, c/o NKK CORPORATION, Tokyo (JP); Tachibana, Yakudo, c/o NKK CORPORATION, Tokyo (JP)
(74) Representative: Grünecker, Kinkeldey, Stockmair & Schwanhäusser Anwaltssozietät

(56) References cited:
- EP-A- 0 002 575
- FR-A- 2 358 924
- US-A- 3 959 181
- US-A- 4 225 732
- US-A- 4 567 157

## Description

### BACKGROUND OF THE INVENTION

This invention relates to catalysts for producing phenol and processes for producing phenol from benzoic acid through vapor phase oxidation in the presence of the catalyst.

Heretofore, various catalysts and processes were developed for producing phenol from benzoic acid through catalytic oxidation in vapor phase. For example, Japanese Patent KOKAI No. 57-11932 discloses a catalyst for producing phenol composed of at least one of copper compounds, vanadium compounds, silver compounds, lithium compounds, sodium compounds and magnesium compounds, and a process for producing phenol using the above catalyst. Another catalyst and process are disclosed in Japanese Patent KOKOKU No. 59-20384. The catalyst contains oxidized copper, zirconium and alkali metal, and the process uses them supported on α-alumina carrier as the catalyst. Moreover, Japanese Patent KOKOKU No. 64-934 (=JP-A-58-32835) discloses a process for producing phenol using an oxide catalyst composed of many kinds of metal elements, i.e. molybdenum as the essential element, at least one of vanadium, niobium and tantalum, at least one of copper, silver, manganese, iron, cobalt, nickel, rhodium, palladium and platinum, and at least one of thallium, alkali metals and alkaline earth metals.

US-A-4 567 157 describes a multicomponent catalyst comprising copper and nickel oxides, suitable for producing phenol from benzoic acid.

However, the catalyst disclosed in Japanese Patent KOKAI No. 57-11932 is insufficient in the yield of phenol. Actually, the maximum conversion of benzoic acid was 50.5 %, and the maximum selectivity to phenol was 88.6 %, and therefore, the maximum yield was 44.7 %. Besides, when an exothermic reaction is conducted such as the oxidation reaction of benzoic aicd using the catalyst containing a copper compound, hot spots occur in the catalyst layer to progress calcining of the catalyst. As a result, the catalytic activity is degraded sharply. In the manufacturing process disclosed in Japanese Patent KOKOKU No. 59-20384, the yield of phenol is insufficient. That is, the maximum conversion of benzoic acid was 63.7 %, and the maximum selectivity to phenol was 82.2 %, and therefore, the maximum yield was 52.4 %. Moreover, byproducts such as diphenyl oxide are produced abundantly, and therefore, a purification process for the produced phenol is necessary. In the manufacturing process disclosed in Japanese Patent KOKOKU No. 64-934, the maximum conversion of benzoic acid was 75 %, and the maximum selectivity to phenol was 89 %. Therefore, the maximum yield was 66.8 %. Moreover, all of the above conventional processes are inferior in productivity due to low space time yield (the production amount of phenol per unit volume of catalyst per unit time), and therefore, they are disadvantageous in the industrial production of phenol.

The present invention is directed towards the use of a catalyst having a high conversion and high selectivity for producing phenol from benzoic acid in a high yield, which catalyst is resistant to a high temperature.

According to the present invention this use is set out in the attached claims.

### DETAILED DESCRIPTION OF THE INVENTION

The nickel compound supported on a metal oxide carrier include oxide, hydroxide, carbonate, nitrate, chloride, carbide, nitride, and sulfide, and oxide is preferred because stable activity can be exercised from the initial stage.

The metal oxide carrier is titania, magnesia, α-alumina, silica gel, zirconia, tin oxide or lanthanum oxide, for example, and titania, magnesia and α-alumina are preferred in view of high conversion and high selectivity.

The conversion of benzoic acid can be improved by adding an alkali metal compound or an alkaline earth metal compound. The alkali metal compound is oxide, carbonate, hydroxide or nitrate, for example, and preferable compounds are oxides such as Li₂O, Na₂O, K₂O, Rb₂O and Cs₂O, and Na₂O and K₂O are particularly preferred in view of the low production of CO and CO₂ and the high selectivity to phenol. The alkaline earth metal compound is also oxide, carbonate, hydroxide or nitrate, for example, and preferable compounds are oxides such as MgO, CaO, SrO and BaO, and CaO is particularly preferred.

A suitable content of the nickel compound is 0.5 to 50 wt. %, preferably 2 to 20 wt. %. When the content is less than 0.5 wt. %, the conversion of benzoic acid is low. While, when the content is beyond 50 wt. %, the production of CO and CO₂ increases by the complete combustion resulting to reduce the selectivity to phenol. A suitable content in the sum of the alkali metal compound and the alkaline earth metal compound is about 0.1 to 30 wt. %, preferably 0.5 to 5 wt. %.

The nickel compound can be supported on the metal oxide carrier by utilizing a conventional impregnation technique. The alkali metal compound and the alkaline earth metal compound may be added to the catalyst before, after or during supporting the nickel compound on the metal oxide carrier.

The catalyst composition prepared by the impregnation is dried in the atmosphere at 90 to 150°C for 12 to 36 hours. The dried matter may be calcined in the atmosphere or a nitrogen gas atmosphere at 400 to 1,000°C for 1 to 10 hours.

The conversion of benzoic acid and the selectivity to phenol can be improved by adding an alkali metal compound, an alkaline earth metal compound or both of them.

Suitable alkali metal compounds and alkaline earth metal compounds and preferred ones are the same as aforementioned.

A suitable content of the alkali metal compound and that of the alkaline earth metal compound are 0.05 to 30 wt. %, preferably 0.1 to 10 wt. %, as the value converted to the oxide thereof, respectively. When the content is less than 0.05 wt. %, the production of CO and CO₂ increases resulting to reduce the selectivity to phenol. While, when the content is beyond 30 wt. %, the conversion of benzoic acid decreases. In the case of adding both of the alkali metal compound and the alkaline earth metal compound, each suitable content of both compounds converted to the oxide thereof is about 0.01 to 20 wt. %, preferably 0.05 to 5 wt. %, respectively.

The nickel oxide catalyst is supported on a metal oxide carrier.

The above nickel oxide catalysts can be prepared according to a known method for preparing conventional catalysts. That is, the raw material nickel oxide may be nitrate, carbonate, organic acid salt, halide, hydroxide or oxide, for example. When the alkali metal compound or the alkaline earth metal compound is incorporated into the catalyst, for example, the alkali metal compound or the alkaline earth metal compound may be added to a gel of nickel hydroxide as it is or in a state of a solution. Then, the mixture is dried and calcined. Besides, the alkali metal compound or the alkaline earth metal compound may be added to the nickel oxide, or may be added to the calcined nickel oxide by blending or impregnation. Furthermore, a mixture of nickel oxide powder and the alkali metal compound powder or the alkaline earth metal compound powder is prepared, and the powder mixture may be pelletized by pressing.

During preparing the nickel oxide catalysts of the invention, it is preferable to calcine the nickel oxide or a precursor thereof. The calcination is conducted at 600 to 900°C, preferably 650 to 850°C in the atmosphere or an inert gas atmosphere so as to crystallize the nickel oxide. In general, when a catalyst prepared by a conventional method is calcined at a temperature higher than about 600°C, the catalytic activity decreases due to the decrease of specific surface area. However, in the case of the nickel oxide catalysts of the invention, phenol-producing activity is improved to obtain a high conversion of benzoic acid and a high selectivity to phenol by the calcination at 600 to 900°C, although the specific surface area is decreased. When the calcination temperature is lower than about 600°C, only CO and CO₂ production proceeds by the complete combustion. Phenol is produced little, and carbonaceous materials deposit on the surface of the catalyst. While, when the calcination temperature is higher than about 900°C, the conversion of benzoic acid is sharply reduced, and the production of phenol is minor.

Phenol is produced from benzoic acid in the presence of at least one of the aforementioned catalyst, and at that time, oxygen gas is supplied together with benzoic acid. The supply amount of oxygen gas is more than the theoretical amount against the supply amount of benzoic acid, and 0.5 to 50 times moles is preferred. When the supply amount of oxygen gas is more than 50 times that of benzoic acid, the complete combustion of benzoic acid frequently occurs. While, when the supply amount of oxygen gas is less than 0.5 times, sufficient conversion of benzoic acid cannot occur. The oxygen gas may be in a form of air or pure oxygen gas, and they may be diluted with an inert gas.

The reaction is, in general, conducted in the presence of water vapor, and the supply amount of water vapor is preferably 1 to 100 times that of benzoic acid in a molar ratio. The supply amount beyond about 100 times is disadvantageous in an economical viewpoint. While, when the supply amount is less than about one time, the selectivity to phenol is degraded. The space velocity of the reaction gas, i.e. the sum of benzoic acid, oxygen-containing gas and water vapor, is preferably 100 to 50,000 hr⁻¹, more preferably 2,000 to 20,000 hr⁻¹, furthermore preferably 5,000 to 20,000 hr⁻¹. When the space velocity is less than about 100 hr⁻¹, the space time yield is insufficient. While, when the space velocity is beyond about 50,000 hr⁻¹, the conversion of benzoic acid is low. The reaction temperature is usually 200 to 600°C, and 350 to 500°C is preferred. When the reaction temperature is higher than about 600°C, the selectivity to phenol decreases. While, when the reaction temperature is lower than about 200 °C, the conversion of benzoic acid is insufficient. The reaction pressure may be any pressure capable of keeping the supplied raw materials in a vapor state, and it is usually ordinary pressure or slightly pressurized condition.

The reaction apparatus may be fixed bed type or fluidized bed type.

The catalysts of the invention exercise a high conversion of benzoic acid and a high selectivity to phenol, and phenol can be produced in a high yield, particularly in a high space time yield through the processes of the invention using the above catalysts.

### EXAMPLES

### Examples 1,2

38.9 g of nickel nitrate (Ni(NO₃)₂·6H₂O) was dissolved in 300 cc of pure water purified by using ion exchange resins, and 188 g of titanium dioxide was added to the solution with stirring. The suspension was evaporated and dried at 120°C. The dried matter was calcined in the atmosphere at 500°C for 3 hours, and crushed into a prescribed mesh. The catalyst powder was impregnated in a potassium hydroxide solution containing 2.4 g of potassium hydroxide dissolved in pure water, and dried at 120°C. The catalyst powder was calcined again in the atmosphere at 500°C for 3 hours to obtain a catalyst composed of nickel oxide, potassium oxide and titanium dioxide in the ratio by weight of 5 : 1 : 94 as the ratio of NiO : K₂O : TiO₂.

### Example 3-5

77.8 g of nickel nitrate (Ni(NO₃)₂·6H₂O) was dissolved in 300 cc of pure water, and 180 g of titanium dioxide was impregnated in the solution with stirring. After drying at 120°C, the dried matter was calcined in the atmosphere at 500°C for 3 hours to obtain a catalyst having a ratio by weight of 10 : 90 as NiO : TiO₂.

### Comparative Example 1

200 g of nickel nitrate was dissolved in 500 ml of pure water, and about 100 g of sodium hydroxide was dissolved in 500 ml of pure water. Both solutions were added dropwise to 2 ℓ of pure water so as to maintain pH 7-8. After adding, the mixture was stirred for about 1 hour. The precipitates produced were filtered and washed. The cake was dried in the atmosphere at 120°C for 24 hours, and then calcined in the atmosphere at 800°C for 4 hours to obtain a catalyst.

### Comparative Example 2

A catalyst was prepared according to Example 1 described in Japanese Patent KOKOKU No. 64-934.

### Comparative Example 3

A catalyst was prepared according to Reference Example 1 described in Japanese Patent KOKOKU No. 59-20384.

### Production of Phenol

Each catalyst was crushed and sieved to a prescribed mesh, and the amount described in Table 1 or 2 was placed in a quartz tube having an inner diameter of 20 mm. Benzoic acid, water vapor, air and nitrogen gas were supplied at the rate described in Table 1 or 2, and allowed to react at the temperature described in Table 1 or 2.

The experimental results are summarized in Table 1 and 2.

**Table 1**

| | Example 1 | Example 2 | Example 3 | Example 4 | Example 5 |
|---|---|---|---|---|---|
| Catalyst Composition (wt.%) | NiO-K₂O-TiO₂ (5:1:94) | NiO-K₂O-TiO₂ (5:1:94) | NiO-TiO₂ (10:90) | NiO-TiO₂ (10:90) | NiO-TiO₂ (10:90) |
| Catalyst Amount (g) | 20 | 20 | 20 | 5 | 5 |
| Reaction Temperature (°C) | 400 | 400 | 400 | 400 | 450 |
| Supply Rate of Benzoic Acid (10⁻⁴mol/min) | 1.45 | 1.45 | 1.45 | 0.55 | 0.55 |
| Supply Rate of Water Vapor (10⁻³mol/min) | 4.35 | 4.35 | 1.45 | 1.10 | 1.10 |
| Supply Rate of Air (cc/min) | 20 | 16 | 32 | 6 | 6 |
| Supply Rate of Nitrogen Gas (cc/min) | 16 | 16 | 49 | 18 | 18 |
| Conversion of Benzoic Acid (%) | 95 | 87 | 54 | 10 | 58 |

| Selectivity (%) | | | | | |
|---|---|---|---|---|---|
| Phenol | 64 | 69 | 73 | 75 | 57 |
| Benzene | 25 | 20 | 20 | 18 | 37 |
| CO, CO₂ | 3 | 1 | 6 | tr | 1 |
| Yield of Phenol (%) | 95x0.64 | 87x0.69 | 54x0.73 | 10x0.75 | 58x0.57 |

**Table 2**

| | Comparative 1 | Comparative 2 | Comparative 3 |
|---|---|---|---|
| Catalyst Composition (wt. ratio) | NiO (100) | MoO₃-V₂O₅-CuO-Na₂O-Al₂O₃ (3.9:3.7:3.8:5.9:82.7) | CuO-ZnO-K₂O-Al₂O₃ (4.0:3.0:3.6:89.4) |
| Catalyst Amount (g) | 5 | 5 | 5 |
| Catalyst Calcining Temperature (°C) | 800 | 600 | 500 |
| Reaction Temperature ( C) | 400 | 300 | 300 |
| Supply Rate of Benzoic Acid (10⁻⁴mol/min) | 2.70 | 2.70 | 2.70 |
| Supply Rate of Water Vapor (10⁻³mol/min) | 8.10 | 8.10 | 8.10 |
| Supply Rate of Air (cc/min) | 12 | 12 | 12 |
| Supply Rate of Nitrogen Gas (cc/min) | 60 | 60 | 60 |
| Conversion of Benzoic Acid (%) | 8 | 48 | 25 |

| Selectivity (%) | | | |
|---|---|---|---|
| Phenol | 15 | 80 | 70 |
| Benzene | 16 | 3 | 5 |
| CO, CO₂ | 68 | 16 | 19 |
| Yield of Phenol (%) | 8x0.15 | 48x0.80 | 25x0.70 |

## Claims

1. The use of a catalyst comprising a nickel compound as essential catalytic component supported on a metal oxide carrier in a process for producing phenol from benzoic acid through vapor phase oxidation, wherein the content of the nickel compound in the catalyst is 0.5 to 50 wt. %, with the proviso that the metal oxide carrier is not iron oxide.

2. The use of claim 1 wherein the nickel compound is nickel oxide and the metal oxide carrier is titania, magnesia or α-alumina.

3. The use of claim 1 or 2 wherein the catalyst further contains at least one alkali metal compound or an alkaline earth metal compound.

4. The use of claim 3 wherein both of the alkali metal compound and alkaline earth metal compound are oxides.

5. The use of claim 3 wherein the alkali metal compound is sodium oxide or potassium oxide and the alkaline earth metal compound is calcium oxide.

6. The use of claim 4 wherein the content of the nickel oxide in the catalyst is 2 to 20 wt. % and the content of the sum of the alkali metal oxide and the alkaline earth metal oxide in the catalyst is 0.5 to 5 wt. %.

## Patentansprüche

1. Verwendung eines Katalysators, umfassend eine Nickelverbindung als wesentlichen katalytischen Bestandteil, die auf einem Metalloxidträger aufgebracht ist, in einem Verfahren zur Herstellung von Phenol aus Benzoesäure durch Dampfphasenoxidation, worin der Gehalt der Nickelverbindung in dem Katalysator 0,5 bis 50 Gew.-% beträgt, mit der Maßgabe, daß der Metalloxidträger kein Eisenoxid ist.

2. Verwendung nach Anspruch 1, worin die Nickelverbindung Nickeloxid ist und der Metalloxidträger ist Titanoxid, Magnesiumoxid oder α-Aluminiumoxid.

3. Verwendung nach Anspruch 1 oder 2, worin der Katalysator weiterhin mindestens eine Alkalimetallverbindung oder eine Erdalkalimetallverbindung enthält.

4. Verwendung nach Anspruch 3, worin sowohl die Alkalimetallverbindung als auch die Erdalkalimetallverbindung Oxide sind.

5. Verwendung nach Anspruch 3, worin die Alkalimetallverbindung Natriumoxid oder Kaliumoxid ist und die Erdalkalimetallverbindung ist Calciumoxid.

6. Verwendung nach Anspruch 4, worin der Gehalt des Nickeloxids in dem Katalysator 2 bis 20 Gew.-% beträgt und der Gehalt der Summe des Alkalimetalloxids und des Erdalkalimetalloxids in dem Katalysator beträgt 0,5 bis 5 Gew.-%.

## Revendications

1. Utilisation d'un catalyseur comprenant un composé de nickel comme composant catalytique essentiel, supporté sur un support d'oxyde métallique dans un procédé destiné à la production de phénol à partir de l'acide benzoïque par l'intermédiaire d'une oxydation en phase vapeur , dans laquelle la teneur du composé de nickel dans le catalyseur est de 0,5 à 50 % en poids, à condition que le support d'oxyde métallique ne soit pas de l'oxyde de fer.

2. Utilisation selon la revendication 1 dans laquelle le composé de nickel est un oxyde de nickel et le support d'oxyde métallique est du dioxyde de titane, de la magnésie ou de l'alumine-α .

3. Utilisation selon la revendication 1 ou 2 dans laquelle le catalyseur contient, de plus, au moins un composé de métal alcalin ou un composé de métal alcalino-terreux.

4. Utilisation selon la revendication 3, dans laquelle le composé de métal alcalin et le composé de métal alcalino-terreux sont tous deux des oxydes.

5. Utilisation selon la revendication 3, dans laquelle le composé de métal alcalin est de l'oxyde de sodium ou de l'oxyde de potassium et le composé de métal alcalino-terreux est de l'oxyde de calcium.

6. Utilisation selon la revendication 4, dans laquelle la teneur de l'oxyde de nickel dans le catalyseur est de 2 à 20 % en poids et la teneur de la somme de l'oxyde de métal alcalin et de l'oxyde de métal alcalino-terreux dans le catalyseur est de 0,5 à 5 % en poids.
